(19) Europäisches Patentamt
European Patent Office
Office européen des brevets

(11) EP 4 545 069 A1

(12) # EUROPEAN PATENT APPLICATION
published in accordance with Art. 153(4) EPC

(43) Date of publication:
**30.04.2025 Bulletin 2025/18**

(21) Application number: **23827269.4**

(22) Date of filing: **22.06.2023**

(51) International Patent Classification (IPC):
***A61K 9/14*** *(2006.01)* ***A61K 9/20*** *(2006.01)*
***A61K 31/721*** *(2006.01)* ***A61K 47/02*** *(2006.01)*
***A61K 47/04*** *(2006.01)* ***A61K 47/10*** *(2017.01)*
***A61K 47/12*** *(2006.01)* ***A61K 47/14*** *(2017.01)*
***A61K 47/18*** *(2017.01)* ***A61K 47/20*** *(2006.01)*
***A61K 47/22*** *(2006.01)* ***A61K 47/28*** *(2006.01)*
***A61K 47/32*** *(2006.01)* ***A61K 47/34*** *(2017.01)*
***A61K 47/36*** *(2006.01)* ***A61K 47/38*** *(2006.01)*
***A61K 47/40*** *(2006.01)* ***A61K 47/42*** *(2017.01)*
***A61P 43/00*** *(2006.01)*

(52) Cooperative Patent Classification (CPC):
**A61K 9/14; A61K 9/20; A61K 31/721; A61K 47/02; A61K 47/10; A61K 47/12; A61K 47/14; A61K 47/18; A61K 47/20; A61K 47/22; A61K 47/28; A61K 47/32; A61K 47/34; A61K 47/36; A61K 47/38;** (Cont.)

(86) International application number:
**PCT/JP2023/023170**

(87) International publication number:
**WO 2023/249087 (28.12.2023 Gazette 2023/52)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **24.06.2022 JP 2022101870**

(71) Applicant: **Sunsho Pharmaceutical Co., Ltd.**
**Fuji-shi, Shizuoka 419-0201 (JP)**

(72) Inventors:
- **MINEDA Misuzu**
  **Fuji-shi, Shizuoka 419-0201 (JP)**
- **OOKAWARA Masaki**
  **Fuji-shi, Shizuoka 419-0201 (JP)**
- **SAKAI Rie**
  **Fuji-shi, Shizuoka 419-0201 (JP)**
- **TODO Hiroaki**
  **Sakado-shi, Saitama 350-0295 (JP)**

(74) Representative: **TBK**
**Bavariaring 4-6**
**80336 München (DE)**

(54) **PHARMACEUTICAL COMPOSITION AND METHOD FOR PRODUCING SAME**

(57) Provided is a pharmaceutical composition or the like having a new form capable of being orally administered even when the pharmaceutical composition contains a polymer component as a medicinal ingredient. A pharmaceutical composition includes: a core particle containing a medicinal ingredient and an absorption enhancing agent; and a coating particle layer coating the core particle.

[FIG. 1]

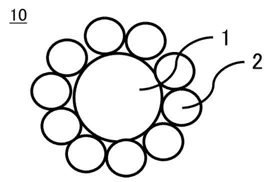


EP 4 545 069 A1

(52) Cooperative Patent Classification (CPC): (Cont.)
**A61K 47/40; A61K 47/42; A61P 43/00**

## Description

### Technical Field

The present invention relates to a pharmaceutical composition and a method for producing the same.

### Background Art

There are various administration routes of pharmaceutical products, and examples of the administration route include oral administration, intravenous administration, intramuscular administration, subcutaneous administration, intradermal administration, cutaneous administration, pulmonary administration, and enteral administration. Among them, oral administration is most often used because a pharmaceutical product can be safely and easily administered.

However, in the case of oral administration, a medicinal ingredient of a pharmaceutical product is usually absorbed in the small intestine via the oral cavity, the esophagus, and the stomach, and thus there has been a tendency that an applicable medicinal ingredient is limited due to, for example, restriction of membrane permeation in the small intestine. In particular, it is difficult to orally administer polymer components such as peptides and proteins, and intravenous administration tends to be selected.

However, in recent years, in order to enhance absorption of a drug in the small intestine or the like, attention has been attracted to a technique of orally administering a polymer component using an absorption enhancing agent.

For example, Patent Literature 1 describes an invention relating to a solid composition containing a GLP-1 peptide and an enhancer for use as a medicament by oral administration. Patent Literature 1 describes that the enhancer (absorption enhancing agent) is a compound that increases the bioavailability of the GLP-1 peptide, and specific examples of the absorption enhancing agent include sodium caprate and SNAC. It is noted that, in Examples of Patent Literature 1, it is described that a tablet composition is prepared by mixing a GLP-1 peptide, an enhancer (absorption enhancing agent), and an additive, and compacting the mixture with a roller.

### Citation List

#### Patent Literature

Patent Literature 1: JP 2016-519128 A

### Summary of Invention

#### Technical Problem

Under such circumstances, there is a demand for a pharmaceutical product in a novel form that can be orally administered even when the pharmaceutical product contains a medicinal ingredient such as a polymer component.

#### Solution to Problem

The present invention includes, for example, the following aspects.

[1] A pharmaceutical composition including:

a core particle containing a medicinal ingredient and an absorption enhancing agent; and
a coating particle layer coating the core particle.

[2] The pharmaceutical composition according to [1], wherein the core particle further contains a thickener.

[3] The pharmaceutical composition according to [1], wherein the core particle further contains a hydrophobic thickener.

[4] The pharmaceutical composition according to [2] or [3], wherein the thickener contains at least one selected from a group consisting of carrageenan, gelatin, glyceryl monostearate, agar, polyethylene glycol, methyl cellulose, hydroxypropyl methyl cellulose, hydroxypropyl cellulose, an aminoalkyl methacrylate copolymer, and an ammonioalkyl methacrylate copolymer.

[5] The pharmaceutical composition according to any one of [1] to [4], wherein the coating particle layer contains at least one selected from a group consisting of bentonite, ethyl cellulose, corn starch, rice starch, wheat starch, potato

starch, calcium stearate, magnesium carbonate, sodium carboxymethyl starch having a low degree of substitution, sodium starch glycolate, anhydrous silicic acid, magnesium silicate, diatomaceous earth, zeolite, silicon dioxide, powdered agar, croscarmellose sodium, crospovidone, and talc.

[6] The pharmaceutical composition according to any one of [1] to [5], wherein the core particle further contains an enteric polymer.

[7] The pharmaceutical composition according to any one of [1] to [6], wherein the absorption enhancing agent contains at least one selected from a group consisting of sodium caprate, sodium caprylate, SNAC, cell membrane penetrating peptide, C-CPE, Angubindin-1, polyoxyethylene alkyl ethers, sodium lauryl sulfate, saponin, chitosan, cyclodextrin, alkyl saccharide, sucrose fatty acid ester, N-acyl amino acid, N-acyl taurine, glycocholic acid, taurocholic acid, deoxycholic acid, ethylenediaminetetraacetic acid (EDTA), sodium salicylate, sodium laurate, tryptophan, arginine, and fatty acids having 8 to 30 carbon atoms.

[8] The pharmaceutical composition according to any one of [1] to [7], wherein a content of the absorption enhancing agent is 1 to 20% by mass with respect to a total mass of the pharmaceutical composition.

[9] The pharmaceutical composition according to any one of [1] to [8], wherein the medicinal ingredient is a polymer component.

[10] The pharmaceutical composition according to any one of [1] to [9], wherein a content of the absorption enhancing agent is 1 to 500 parts by mass with respect to 1 part by mass of the medicinal ingredient.

[11] A method for producing the pharmaceutical composition according to any one of [1] to [10], comprising a granulation step of inputting a droplet of a granulation solution containing a medicinal ingredient and an absorption enhancing agent into a fluidized bed in which coating particles flow, and performing granulation by attaching the coating particles to a surface of the droplet.

## Advantageous Effects of Invention

According to the present invention, there is provided a pharmaceutical composition or the like in a novel form that can be orally administered even when the pharmaceutical composition contains a medicinal ingredient such as a polymer component.

## Brief Description of Drawings

[Fig. 1] Fig. 1 is a schematic view of a pharmaceutical composition according to an embodiment of the present invention.

## Description of Embodiments

Hereinafter, embodiments for carrying out the present invention will be described in detail.

### 1 Pharmaceutical Composition

A pharmaceutical composition according to one aspect of the present invention contains: a core particle containing a medicinal ingredient and an absorption enhancing agent; and a coating particle layer coating the core particle.

The pharmaceutical composition according to one aspect of the present invention can be orally administered even when it contains a polymer component as a medicinal ingredient. As a result, at least one of effects such as high safety, high compliance due to easy administration, and low manufacturing costs can be obtained.

Hereinafter, the present invention will be described with reference to the drawings. It is noted that the drawings may be exaggerated for the sake of explanation and may be different from actual dimensions.

Fig. 1 is a schematic view of a pharmaceutical composition according to an embodiment of the present invention.

A pharmaceutical composition 10 includes a core particle 1 containing a medicinal ingredient and an absorption enhancing agent, and a coating particle layer 2 coating the core particle 1. By having the coating particle layer 2 formed by attaching coating particles to the surface of the core particle 1, the core particle 1 can be granulated into the shape of the pharmaceutical composition 10.

In the embodiment of the present invention, when a solid preparation (for example, a tablet) containing the pharmaceutical composition 10 is taken, at least a part of the coating particle layer 2 is peeled off or dissolved through the oral cavity, esophagus, and stomach, and the core particle 1 is exposed when reaching the small intestine. Here, the medicinal ingredient contained in the core particle 1, for example, insulin (peptide hormone) has a large molecular weight and a surface charge, and thus is usually hardly absorbed in the small intestine. However, such a medicinal ingredient is easily absorbed in the small intestine in the presence of an absorption enhancing agent. Although the specific mechanism of action thereof is not necessarily clear, examples thereof include absorption by an intercellular space pathway based on

an opening of an intercellular space and absorption by an intracellular pathway based on cell membrane perturbation. As a result, even when the pharmaceutical composition 10 contains a polymer component such as insulin as a medicinal ingredient, the polymer component is suitably absorbed from the small intestine or the like by oral administration, and bioavailability of the pharmaceutical composition 10 can be increased.

It is noted that, in one embodiment of the present invention, the medicinal ingredient may be absorbed in an organ other than the small intestine, for example, the stomach, and a mode in which the medicinal ingredient is absorbed in the stomach in the presence of an absorption enhancing agent is also included in the present invention. In another embodiment, the medicinal ingredient may be absorbed in the large intestine, and a mode in which the medicinal ingredient is absorbed in the large intestine in the presence of an absorption enhancing agent is also included in the present invention.

The average particle diameter of the pharmaceutical composition is preferably 10 to 1,000 μm, and more preferably 100 to 500 μm. When the average particle diameter of the pharmaceutical composition is 10 μm or more, sufficient hardness can be obtained, and the shape thereof can be preferably maintained in a preparation step such as tableting. On the other hand, when the average particle diameter of the pharmaceutical composition is 1,000 μm or less, a tablet or the like to be obtained can be easily taken, which is preferable. It is noted that, in the present specification, the "particle diameter" means the maximum distance among distances between two points on the contour line of an object. In addition, the "average particle diameter" means an average value of particle diameter of any 50 objects included in one field of view of a scanning electron microscope (SEM).

Hereinafter, each configuration of the composition of the present invention will be described in detail.

[Core Particle]

A core particle contains a medicinal ingredient and an absorption enhancing agent. The core particle may further contain a thickener, an enteric polymer, a pH adjusting agent, a solvent, an additive, and the like.

(Medicinal Ingredient)

The medicinal ingredient is not particularly limited, but is more preferably a polymer component. It is noted that, in the present specification, the term "polymer" means that the molecular weight is 500 or more, preferably 500 to 1,000,000, more preferably 500 to 200,000, and still more preferably 500 to 10,000.

Examples of the polymer component include antibodies, peptides, proteins, polymeric substances, nucleic acids, and biosimilars thereof.

Specific examples of the polymer component include cytokines such as insulin, calcitonin, angiotensin, vasopressin, desmopressin, LH-RH (luteinizing hormone-releasing hormone), somatostatin, glucagon, oxytocin, gastrin, cyclosporin, somatomedin, secretin, h-ANP (human atrial natriuretic peptide), ACTH (adrenocorticotropic hormone), MSH (melanocyst stimulating hormone), β-endorphin, muramyl dipeptide, enkephalin, neurotensin, bombesin, VIP (vasoactive intestinal peptide), CCK-8 (cholecystokinin-8), PTH (parathyroid hormone), CGRP (calcitonin gene-related peptide), TRH (thyrotropin releasing hormone), endothelin, hGH (human growth hormone), interleukin, interferon, colony stimulating factor, and tumor necrosis factor, GLP-1 receptor agonists (semaglutide), and cyanocobalamin.

One kind of these medicinal ingredient may be used alone, or two or more kinds thereof may be used in combination.

In one embodiment, the content of the medicinal ingredient is preferably 0.01 to 10% by mass, preferably 0.01 to 5% by mass, more preferably 0.03 to 3% by mass, still more preferably 0.05 to 1% by mass, and particularly preferably 0.15 to 0.8% by mass, with respect to the total mass of the pharmaceutical composition.

In another embodiment, the content of the medicinal ingredient is preferably 0.1 to 10% by mass, more preferably 0.4 to 10% by mass, still more preferably 0.4 to 5% by mass or 1 to 10% by mass, particularly preferably 1 to 5% by mass, and most preferably 1.5 to 4% by mass, with respect to the total mass of the pharmaceutical composition.

(Absorption Enhancing Agent)

The absorption enhancing agent is not particularly limited as long as it is a component having a function of enhancing absorption of the medicinal ingredient, but has, for example, a function of enhancing absorption of the medicinal ingredient by acting on a cell membrane of the small intestine or the like and an intercellular space or the like.

The absorption enhancing agent is not particularly limited, but preferably contains at least one selected from the group consisting of sodium caprate, sodium caprylate, SNAC, cell membrane penetrating peptide, C-CPE, Angubindin-1, polyoxyethylene alkyl ethers, sodium lauryl sulfate, saponin, chitosan, cyclodextrin, alkyl saccharide, sucrose fatty acid ester, N-acyl amino acid, N-acyl taurine, glycocholic acid, taurocholic acid, deoxycholic acid, ethylenediaminetetraacetic acid (EDTA), sodium salicylate, sodium laurate, tryptophan, arginine, and fatty acid with 8 to 30 carbon atoms, more

preferably contains at least one selected from the group consisting of sodium caprate, sodium caprylate, SNAC, cell membrane penetrating peptide, C-CPE, and Angubindin-1, and still more preferably contains sodium caprate and/or SNAC.

"SNAC" is also called sodium salcaprozate and has the following structure.

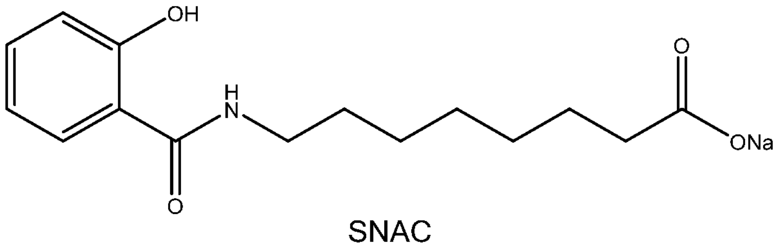

SNAC

"C-CPE" includes C-terminal fragments of Clostridium perfringens enterotoxin and variants thereof, and examples thereof include C-CPE184-319, C-CPE194-319, and C-CPE Y306W/S313H.
"Angubindin-1" is an amino acid region of 421 to 644 of a component Ib of two proteins (component Ia and component Ib) constituting iota toxin produced by Clostridium perfringens.
Examples of polyoxyethylene alkyl ethers include polyoxyethylene caprylic ether, polyoxyethylene lauryl ether, polyoxyethylene myristyl ether, and polyoxyethylene stearyl ether.
Examples of alkyl saccharide include β-octyl glucoside, β-decyl glucoside, β-decyl galactoside, β-octylmannoside, β-dodecyl maltoside, β-dodecyl isomaltoside, β-dodecyl maltotrioside, β-stearyl glucoside, β-stearyl galactoside, β-stearyl mannoside, β-stearyl maltoside, β-stearyl isomaltoside, decyl oligoglucoside, and dodecyl oligoglucoside.
Examples of N-acyl amino acid include those obtained by N-acylating an amino acid with a fatty acid having 8 to 30 carbon atoms to be described later. The amino acid is not particularly limited, but is preferably glutamic acid, aspartic acid, glycine, or alanine. The fatty acid is not particularly limited, but is preferably caprylic acid, capric acid, lauric acid, myristic acid, palmitic acid, or stearic acid, and more preferably lauric acid.
Examples of sucrose fatty acid ester include a sucrose fatty acid ester obtained by esterifying a hydroxy group of sucrose with a fatty acid having 8 to 30 carbon atoms to be described later. At this time, the fatty acid is not particularly limited, but is preferably caprylic acid, capric acid, lauric acid, myristic acid, palmitic acid, or stearic acid, and more preferably lauric acid. The sucrose fatty acid may be a monoester, may be a diester, may be a triester, may be a tetraester, or may be a mixture thereof. The HLB (hydrophilic-lipophilic balance) of the sucrose fatty acid ester is preferably 1 to 18, and more preferably 3 to 15.
Examples of fatty acids having 8 to 30 carbon atoms include saturated fatty acids having 8 to 30 carbon atoms and unsaturated fatty acids having 8 to 30 carbon atoms. The saturated fatty acid having 8 to 30 carbon atoms is not particularly limited, and examples thereof include caprylic acid, capric acid, lauric acid, myristic acid, palmitic acid, stearic acid, arachidic acid, and behenic acid. The unsaturated fatty acid having 8 to 30 carbon atoms is not particularly limited, and examples thereof include oleic acid, linoleic acid, α-linolenic acid, γ-linolenic acid, and arachidonic acid.
One kind of the above-described absorption enhancing agents may be used alone, or two or more kinds thereof may be used in combination.
In one embodiment, with respect to the total mass of the pharmaceutical composition, the content of the absorption enhancing agent is preferably 0.01 to 80% by mass, and more preferably 0.01 to 50% by mass, 0.01 to 30% by mass, 0.01 to 20% by mass, 0.01 to 15% by mass, 0.01 to 10% by mass, 0.1 to 50% by mass, 0.1 to 30% by mass, 0.1 to 20% by mass, 0.1 to 15% by mass, 0.1 to 10% by mass, 0.3 to 50% by mass, 0.3 to 30% by mass, 0.3 to 20% by mass, 0.3 to 15% by mass, 0.3 to 10% by mass, 1 to 50% by mass, 1 to 30% by mass, 1 to 20% by mass, 1 to 10% by mass, 3 to 50% by mass, 3 to 30% by mass, 3 to 20% by mass, 3 to 10% by mass, 5 to 50% by mass, 5 to 30% by mass, 5 to 20% by mass, 5 to 10% by mass, 10 to 50% by mass, 10 to 30% by mass, 10 to 20% by mass, 15 to 50% by mass, 20 to 50% by mass, or 30 to 50% by mass. According to the pharmaceutical composition of the present invention, the content of the absorption enhancing agent can be widely set due to the shape retention function by the coating particle layer, and for example, the shape of the pharmaceutical composition can be suitably retained even if a large amount of the absorption enhancing agent is contained.
In another embodiment, the content of the absorption enhancing agent is preferably 1 to 80% by mass, more preferably 1 to 20% by mass, still more preferably 2 to 20% by mass, and particularly preferably 3 to 10% by mass, with respect to the total mass of the pharmaceutical composition. It is preferable that the content of the absorption enhancing agent is 1% by mass or more from the viewpoint that bioavailability can be increased. On the other hand, when the content of the absorption enhancing agent is 80% by mass or less, the contents of other components (medicinal ingredient,

thickener, enteric polymer, and the like) can be increased, which is preferable from the viewpoint of enabling various preparation designs.

Further, according to the pharmaceutical composition of the present invention, the content (ratio) of the absorption enhancing agent to the medicinal ingredient can be increased, and absorption of a drug can be enhanced as the content (ratio) of the absorption enhancing agent increases (refer to Buckley et al., Sci. Transl. Med. 10, eaar7047 (2018) 14 and the like).

In addition, the content of the absorption enhancing agent is preferably 1 to 500 parts by mass, more preferably 1 to 300 parts by mass, and still more preferably 1 to 200 parts by mass, 1 to 100 parts by mass, 1 to 70 parts by mass, 1 to 60 parts by mass, 1 to 50 parts by mass, 1 to 20 parts by mass, 1 to 10 parts by mass, 1 to 5 parts by mass, 10 to 100 parts by mass, 20 to 100 parts by mass, 50 to 200 parts by mass, 60 to 200 parts by mass, 70 to 200 parts by mass, and 100 to 200 parts by mass, with respect to 1 part by mass of the medicinal ingredient.

(Thickener)

According to one embodiment of the present invention, the core particle further contains a thickener. The thickener may have, for example, a function of stabilizing the shape of the core particle, a function of adjusting the physical properties (hardness and the like) of the pharmaceutical composition, a function of suppressing decomposition of the medicinal ingredient, a function of enhancing membrane permeation of the medicinal ingredient in the small intestine and the like.

Examples of the thickener include a hydrophobic thickener and a hydrophilic thickener. It is noted that, in the present specification, the term "hydrophobic thickener" means that solubility is less than 100 mg/mL when 1 g of the thickener is added to 25 mL of water at 25°C. Further, the term "hydrophilic thickener" means that solubility is 100 mg/mL or more when 1 g of the thickener is added to 25 mL of water at 25°C.

The hydrophobic thickener is not particularly limited, and examples thereof include glyceryl monostearate, an aminoalkyl methacrylate copolymer, and an ammonioalkyl methacrylate copolymer.

The hydrophilic thickener is not particularly limited, and examples thereof include carrageenan, gelatin, agar, polyethylene glycol, methyl cellulose, hydroxypropyl methyl cellulose, and hydroxypropyl cellulose.

In one embodiment, the thickener preferably contains the hydrophobic thickener, and more preferably contains glyceryl monostearate. The hydrophobic thickener can improve bioavailability by enhancing membrane permeation of the medicinal ingredient in the small intestine or the like.

In one embodiment, the thickener preferably contains carrageenan. Carrageenan can swell at least partially when it comes into contact with water in the oral cavity, esophagus, stomach, and small intestine. As a result, for example, the bioavailability can be improved by exhibiting an action of suppressing decomposition of the medicinal ingredient by a digestive enzyme, gastric acid, or the like, a sustained release action of the medicinal ingredient in the small intestine, and the like.

In one embodiment, the thickener preferably contains at least one selected from the group consisting of carrageenan, gelatin, glyceryl monostearate, agar, polyethylene glycol, methyl cellulose, hydroxypropyl methyl cellulose, hydroxypropyl cellulose, an aminoalkyl methacrylate copolymer, and an ammonioalkyl methacrylate copolymer, more preferably contains at least one selected from the group consisting of carrageenan, gelatin, glyceryl monostearate, agar, and polyethylene glycol, and still more preferably contains at least one selected from the group consisting of carrageenan, gelatin, and glyceryl monostearate.

It is noted that one kind of the above-described thickeners may be used alone, or two or more kinds thereof may be used in combination.

The content of the thickener is preferably 1 to 85% by mass, more preferably 1 to 75% by mass, and still more preferably 5 to 65% by mass or more, with respect to the total mass of the pharmaceutical composition. When the content of the thickener is 1% by mass or more, the shape of the thickener can be easily maintained until granulation is performed after ejection, which is preferable. On the other hand, when the content of the thickener is 85% by mass or less, the granulation solution can have a preferable viscosity suitable for ejection.

(Enteric Polymer)

According to the embodiment of the present invention, the core particle further contains an enteric polymer. The enteric polymer can have, for example, a function of suppressing decomposition of the medicinal ingredient in the stomach by including at least a part of the medicinal ingredient in the core particle. It is noted that, in the present specification, the "enteric polymer" means a polymer having a property of not dissolving in the stomach but dissolving in the intestine. In addition, the term "enteric" means a small intestine enteric property and/or a large intestine enteric property, and is preferably a small intestine enteric property.

The enteric polymer is not particularly limited, and examples thereof include a methacrylic acid copolymer,

hydroxypropyl methyl cellulose phthalate (HPMCP), hydroxypropyl methyl cellulose acetate succinate (HPMCAS), polyvinyl acetate phthalate, cellulose acetate phthalate (ceracefate), an ethyl acrylate-methyl methacrylate copolymer, carboxymethyl ethyl cellulose (CMEC), cellulose acetate phthalate (CAP), and polyvinyl alcohol phthalate (PVAP). Among them, the enteric polymer preferably contains at least one selected from the group consisting of a methacrylic acid copolymer, HPMCP, and CMEC, and more preferably contains a methacrylic acid copolymer. It is noted that one kind of the above-described enteric polymers may be used alone, or two or more kinds thereof may be used in combination.

The content of the enteric polymer is preferably 20% by mass or less, more preferably 15% by mass or less, and still more preferably 0.1 to 10% by mass, with respect to the total mass of the pharmaceutical composition.

(PH Adjusting Agent)

The core particle may further contain a pH adjusting agent. The pH adjusting agent has a function of improving the stability of the medicinal ingredient by controlling pH in the core particle.

The pH adjusting agent is not particularly limited, and examples thereof include lactic acid, acetic acid, acetates (sodium acetate and the like), citric acid, citrates (sodium citrate and the like), phosphoric acid, phosphates (sodium phosphate and the like), diisopropanolamine, triisopropanolamine, triethanolamine, potassium hydroxide, sodium hydroxide, and L-arginine. One kind of these pH adjusting agents may be used alone, or two or more kinds thereof may be used in combination.

(Solvent)

The core particle may include a solvent. The solvent has a function of adjusting dispersibility of the medicinal ingredient, hardness of the pharmaceutical composition, and the like.

Examples of the solvent include water and an organic solvent.

The organic solvent is not particularly limited, and examples thereof include propylene glycol monocaprylate, propylene glycol dicaprylate, propylene glycol dicaprate, propylene glycol monolaurate, propylene glycol monooleate, benzyl benzoate, octyldecyl triglyceride, oleic acid, triethyl citrate, dimethylpolysiloxane, cinnamaldehyde, medium-chain monodiglyceride, medium-chain fatty acid triglyceride, triacetin, piperonyl butoxide, diethyl phthalate, dibutyl phthalate, butyl phthalyl butyl glycolate, octyldodecyl myristate, ethyl butyrate, ethanol, and isopropanol.

Among them, solvent preferably contains water. It is noted that one kind of the above-described solvents may be used alone, or two or more kinds thereof may be used in combination.

The content of the solvent is preferably 20% by mass or less, more preferably 0.01 to 15% by mass, and still more preferably 0.1 to 10% by mass, with respect to the total mass of the pharmaceutical composition.

(Additive)

The core particle may contain an additive.

The additive is not particularly limited, and examples thereof include a colorant, a masking agent, a sweetener, an antioxidant, a preservative, a lubricant, and a perfume. One kind of the additives may be used alone, or two or more kinds thereof may be used in combination.

The average particle diameter of the core particles is preferably 5 to 1000 μm, more preferably 10 to 800 μm, and still more preferably 100 to 500 μm. When the average particle diameter of the core particle is 5 μm or more, the ratio of the core particle can be preferably increased. On the other hand, when the average particle diameter of the core particles is 1000 μm or less, a tablet or the like to be obtained can have a preferable size allowing the tablet to be easily taken.

The content of the core particle is preferably 90% by mass or less, and more preferably 10 to 90% by mass, with respect to the total mass of the pharmaceutical composition. When the content of the core particles is 90% by mass or less, the shape stability of the pharmaceutical composition can be improved, which is preferable.

[Coating Particle Layer]

The coating particle layer of the composition of the present invention coats a core particle. The coating particle layer contains coating particles. The coating particles adhere to the surface of the core particle to form a layered form, thereby forming a coating particle layer. The coating particle layer of the present invention is a layer shape, but includes a form completely coating the core particle and a form partially coating the core particle. In the form in which a part of the core particle is coated by the coating particle layer, a part of the core particle not coated with the coating particle layer is exposed.

The coating particle is not particularly limited, but is usually a water-insoluble particle. Specific examples of the coating particle include a divalent or higher metal salt, an inorganic substance, a polysaccharide or a derivative thereof, and an organic polymer.

Examples of the divalent or higher metal salt include calcium chloride, calcium carbonate, calcium oxide, calcium sulfate, calcium hydrogen phosphate, calcium dihydrogen phosphate, calcium stearate, magnesium chloride, magnesium carbonate, magnesium oxide, magnesium silicate, aluminum chloride, aluminum hydroxide, aluminum phosphate, aluminum sulfate, aluminum silicate, potassium alum, iron chloride alum, ammonium alum, ferric sulfate, iron citrate, zinc oxide, zinc sulfate, and hydrates thereof.

Examples of the inorganic substance include anhydrous silicic acid, silicon dioxide, titanium oxide, kaolin, diatomaceous earth, bentonite, zeolite, and talc.

Examples of the polysaccharide or the derivative thereof include corn starch, rice starch, wheat starch, potato starch, powdered agar, crystalline cellulose, ethyl cellulose, croscarmellose sodium, sodium carboxymethyl starch having a low degree of substitution, and sodium starch glycolate.

Examples of the organic polymer include crospovidone, an aminoalkyl methacrylate copolymer E, a methacrylic acid copolymer L, a dry methacrylic acid copolymer LD, a methacrylic acid copolymer S, and an ammonioalkyl methacrylate copolymer.

Among then, the coating particle layer preferably contains, as coating particles, at least one selected from the group consisting of bentonite, ethyl cellulose, corn starch, rice starch, wheat starch, potato starch, calcium stearate, magnesium carbonate, sodium carboxymethyl starch having a low degree of substitution, sodium starch glycolate, anhydrous silicic acid, magnesium silicate, diatomaceous earth, zeolite, silicon dioxide, powdered agar, croscarmellose sodium, crospovidone, and talc, more preferably contains bentonite and/or corn starch, and still more preferably contains bentonite. It is noted that, for example, bentonite can enhance swelling of carrageenan by acting on carrageenan as a thickener present in the core particle. Thereby, the pharmaceutical composition may preferably exhibit an enteric property, more preferably a small intestine property. In one embodiment, the pharmaceutical composition preferably contains a core particle containing a medicinal ingredient, an absorption enhancing agent, and carrageenan, and a coating particle layer coating the core particle and containing bentonite. One kind of the above-described coating particles may be used alone, or two or more kinds thereof may be used in combination.

The average particle diameter of the coating particles is preferably 1 to 100 μm, more preferably 2 to 50 μm, and still more preferably 3 to 30 μm. When the average particle diameter of the coating particles is 1 μm or more, fluidity of the coating particles can be preferably increased. On the other hand, when the average particle diameter of the coating particles is 100 μm or less, the surface area is small, and bonding strength with the coating particles can be preferably increased.

In one embodiment, the average particle diameter of the coating particles is preferably 30 to 100 μm, more preferably 30 to 80 μm, still more preferably 30 to 70 μm, and particularly preferably 40 to 60 μm from the viewpoint of improving absorbability by allowing the core particles to suitably come into contact with a digestion liquid (saliva, gastric acid, intestinal fluid, pancreatic fluid, and the like) due to an increase in voids between the particles.

The content of the coating particle layer is preferably 90% by mass or less, more preferably 10 to 85% by mass, still more preferably 15 to 70% by mass, and particularly preferably 20 to 70% by mass, with respect to the total mass of the pharmaceutical composition. When the content of the coating particle layer is 90% by mass or less, the shape stability of the pharmaceutical composition can be preferably improved.

## 2 Method for Producing Pharmaceutical Composition

According to one aspect of the present invention, a method for producing a pharmaceutical composition is provided. The production method of the present invention includes a granulation step of inputting a droplet of a granulation solution containing a medicinal ingredient and an absorption enhancing agent into a fluidized bed in which coating particles flow, and performing granulation by attaching the coating particles to the surface of the droplet. When the coating particles are attached to the surface of the droplet of the granulation solution, a component (thickener, solvent, or the like) of the granulation solution is absorbed by the coating particles, whereby granulation can be performed.

### [Granulation Step]

The granulation step can use, for example, a granulation method disclosed in JP 2019-073459 A. Specifically, the granulation step is a step in which a droplet of a granulation solution containing a medicinal ingredient and an absorption enhancing agent is put into a fluidized bed in which coating particles flow, and the coating particles are attached to the surface of the droplet to perform granulation. More specifically, it is possible to intermittently eject a predetermined amount of the granulation solution at a time, to put a droplet of the granulation solution into a fluidized bed composed of coating particles, to attach the coating particles to the surface of the droplet, and to allow the component (thickener, solvent, or the like) to be absorbed by the coating particles.

(Granulation Solution)

The granulation solution contains a medicinal ingredient and an absorption enhancing agent. The granulation solution may further contain a thickener, an enteric polymer, a pH adjusting agent, a solvent, an additive, and the like. The granulation solution may be included in the core particle of the pharmaceutical composition.
Each of the components such as a medicinal ingredient, an absorption enhancing agent, a thickener, an enteric polymer, a pH adjusting agent, a solvent, and an additive is as described above.
The content of each of the components such as a medicinal ingredient, an absorption enhancing agent, a thickener, an enteric polymer, a pH adjusting agent, a solvent, and an additive is preferably adjusted to such an amount that the content of the pharmaceutical composition in the core particle falls within the above-described range. It is noted that the content of the core particles in the pharmaceutical composition and the average particle diameter of the core particles can be adjusted by adjusting the content of a solid content in the granulation solution (total content of components other than the solvent in the granulation solution).
In one embodiment, the granulation solution preferably contains water. At this time, the content of water is preferably 60 to 98% by mass, more preferably 70 to 95% by mass, and still more preferably 80 to 90% by mass, with respect to the total mass of the granulation solution. When the content of water is within the above-described range, the state is preferable because the form of the pharmaceutical composition can be suitably formed when a droplet of the granulation solution is put into a fluidized bed composed of coating particles described later.
A viscosity of the granulation solution is not particularly limited, but is preferably 0.5 to 20,000 mPa, and more preferably 1 to 10,000 mPa. When a viscosity of the granulation solution is 0.5 mPa or more, the viscosity is preferable because it is easy to maintain the shape when the droplet of the ejected granulation solution is put into the fluidized bed. When the viscosity of the granulation solution is 20,000 mPa or less, productivity is preferably excellent.

(Ejection of Granulation Solution)

The granulation solution is preferably ejected intermittently by a predetermined amount. At this time, the ejected granulation solution has a droplet shape. The ejection of the granulation solution can be performed by, for example, a liquid agent quantitative discharge device including a discharge nozzle.
The ejection amount per one time of the granulation solution can be usually controlled by the nozzle diameter and the discharge pressure of the discharge nozzle of the liquid agent quantitative discharge device. It is noted that the average particle diameter of the core particles can be controlled by adjusting the ejection amount of the granulation solution.
A nozzle diameter of the discharge nozzle is not particularly limited, but is preferably 0.1 to 1.0 mm, and more preferably 0.2 to 0.5 mm. When a nozzle diameter of the discharge nozzle is 0.1 mm or more, the nozzle diameter is preferable from the viewpoint of being able to discharge even a highly viscous granulation solution, excellent productivity, and the like. On the other hand, when the nozzle diameter of the discharge nozzle is 1.0 mm or less, the shape of the droplet can be preferably maintained when the solution droplet comes into contact with the fluidized bed.
A discharge pressure is not particularly limited, but is preferably 0.1 to 5.0 MPa, and more preferably 0.1 to 2.0 MPa. When a discharge pressure is 0.1 MPa or more, the discharge pressure is preferable from the viewpoint of being able to discharge even a highly viscous granulation solution, excellent productivity, and the like. On the other hand, when the discharge pressure is 5.0 MPa or less, the shape of the droplet can be preferably maintained when the solution droplet comes into contact with the fluidized bed.
The time (intermittent time) of an ejection interval of the granulation solution is usually controlled by the number of times of intermittent ejection of the liquid agent quantitative discharge device. The number of times of intermittent ejection is not particularly limited, but is preferably 500 to 50,000 rpm, and more preferably 1000 to 20,000 rpm. When the number of times of intermittent ejection is 500 rpm or more, productivity can be preferably enhanced. On the other hand, when the number of times of intermittent ejection is 50,000 rpm or less, heat generation of the device due to friction can be preferably suppressed. It is noted that, in the present specification, the term "the number of times of intermittent ejection" means the number of times of ejection per minute.

(Input to Fluidized Bed)

A droplet of the ejected granulation solution is introduced into a fluidized bed. Coating particles are accommodated in the fluidized bed, and the coating particles adhere to the surface of the droplet as the droplet comes into contact with the coating particles in the fluidized bed. Subsequently, the attached coating particles absorb the component (thickener, solvent, or the like) in the droplet, so that the fluidity of the droplet is reduced to form core particles. In addition, the coating particles attached to the core particle form a layer structure, thereby forming a coating particle layer. Thus, a pharmaceutical composition can be produced. It is noted that the coating particle is as described above.
Examples of the fluidized bed include a bed in which coating particles are accommodated in a pan granulator and

are rolled. Since a rotating pan (dish) provides a rolling operation to the coated particles, the coated particles can have a layered configuration when the droplet comes into contact with the coating particles. In addition, collision with a subsequent droplet can be prevented or suppressed.

Vibration applied to the coating particle in the fluidized bed is not particularly limited, but is preferably 10 to 500 rpm, and more preferably 20 to 200 rpm. When the vibration is 10 rpm or more, collision with a subsequent droplet can be preferably prevented or suppressed. On the other hand, when the vibration is 500 rpm or less, the coating particle can be preferably suppressed from being biased by centrifugal force.

## 3 Solid Preparation

According to one aspect of the present invention, solid preparation containing a pharmaceutical composition is provided. The dosage form of the solid preparation is not particularly limited, and examples thereof include a tablet, a capsule, a powder, a fine granule, and a granule. Among them, the solid preparation is preferably a tablet.

The tablet is usually formed by compressing a pharmaceutical composition. At this time, an excipient, a disintegrant, a binder, a lubricant, a stabilizer, a suspending agent, a coating agent, an antioxidant, a dispersant, a fluidizing agent, a perfume, a sweetening agent, a flavoring agent, a coloring agent, and the like may be added together with the pharmaceutical composition, and compression molding may be performed.

It is noted that the solid preparation is preferably a preparation for oral administration.

## Examples

Hereinafter, the present invention will be specifically described with reference to Examples, but the present invention is not limited thereto.

### [Example 1]

Glyceryl monostearate as a thickener was heated and dissolved at 80°C, and then fluorescein isothiocyanate-dextran 4 (FITC-dextran4: FD-4) and sodium sarcaprozate (SNAC) each serving as an absorption enhancing agent were added thereto to prepare a granulation solution.

It is noted that FD-4 is dextran (4kDa) labeled with a high-molecular fluorescent substance (fluorescein isothiocyanate), and is a compound for evaluating intestinal permeability having a property of being absorbed by a short intercellular space pathway. The compound is used in relation to evaluation to be described later, and is handled as a medicinal ingredient in the present example.

The prepared granulation solution was charged into a liquid agent quantitative discharge device (discharge nozzle diameter: 0.3 mm) while being heated at 80°C, the discharge pressure was set to 0.5 MPa, and the number of times of intermittent ejection was set to 10,000 rpm.

An open pan granulator (pan diameter: 300 mm) was installed 70 cm below the discharge nozzle, and bentonite (average particle diameter: 50 μm) was accommodated in the open pan granulator. The open pan granulator revolved and vibrated at 90 rpm so as to form a fluidized bed in which bentonite rolls and flows.

The granulation solution was intermittently ejected from the liquid agent quantitative discharge device, and a droplet of the granulation solution was put into the fluidized bed. Bentonite is attached to the surface of the droplet, thereby producing a pharmaceutical composition having a core particle derived from the granulation solution and a coating particle layer of the bentonite. It is noted that the pharmaceutical composition of the open pan granulator and bentonite were separated from each other by a sieve (mesh size: 106 μm).

In the produced pharmaceutical composition, the content of the core particle was 82.5% by mass with respect to the total mass of the pharmaceutical composition, and the content of the coating particle layer was 17.5% by mass with respect to the total mass of the pharmaceutical composition. In addition, the average particle diameter of the pharmaceutical compositions was 230 μm, and the average particle diameter of the core particles was 216 μm.

### [Example 2]

Gelatin and I-carrageenan each serving as a thickener, a methacrylic acid copolymer S serving as an enteric polymer, and water were heated and dissolved at 90°C, then the temperature was lowered to 70°C, and then FD-4 and SNAC serving as an absorption enhancing agent were added, thereby preparing a granulation solution.

A pharmaceutical composition having a core particle derived from the granulation solution and a coating particle layer of bentonite was produced in the same manner as in Example 1.

In the produced pharmaceutical composition, the content of the core particle was 31.3% by mass with respect to the total mass of the pharmaceutical composition, and the content of the coating particle layer was 68.7% by mass with

respect to the total mass of the pharmaceutical composition. In addition, the average particle diameter of the pharmaceutical compositions was 300 μm, and the average particle diameter of the core particles was 204 μm.

[Example A]

A granulation solution was produced in the same manner as in Example 2 except that the contents of FD-4, SNAC, gelatin, l-carrageenan, and methacrylic acid copolymer S were changed.
A pharmaceutical composition having a core particle derived from the granulation solution and a coating particle layer of bentonite was produced in the same manner as in Example 1.
In the produced pharmaceutical composition, the content of the core particle was 29.1% by mass with respect to the total mass of the pharmaceutical composition, and the content of the coating particle layer was 70.9% by mass with respect to the total mass of the pharmaceutical composition. In addition, the average particle diameter of the pharmaceutical compositions was 277 μm, and the average particle diameter of the core particles was 247 μm.

[Example B]

A granulation solution was prepared in the same manner as in Example 2 except that the contents of FD-4, SNAC, gelatin, l-carrageenan, and methacrylic acid copolymer S were changed.
A pharmaceutical composition having a core particle derived from a granulation solution and a coating particle layer of corn starch were produced in the same manner as in Example 1 except that the coating particles was changed to corn starch (average particle diameter: 20 μm).
In the produced pharmaceutical composition, the content of the core particle was 27.1% by mass with respect to the total mass of the pharmaceutical composition, and the content of the coating particle layer was 72.9% by mass with respect to the total mass of the pharmaceutical composition. In addition, the average particle diameter of the pharmaceutical compositions was 256 μm, and the average particle diameter of the core particles was 230 μm.

[Comparative Example 1]

A pharmaceutical composition was produced in the same manner as in Example 1 except that SNAC was not added to the granulation solution.
In the produced pharmaceutical composition, the content of the core particle was 82.5% by mass with respect to the total mass of the pharmaceutical composition, and the content of the coating particle layer was 17.5% by mass with respect to the total mass of the pharmaceutical composition. In addition, the average particle diameter of the pharmaceutical compositions was 230 μm, and the average particle diameter of the core particles was 216 μm.

[Comparative Example 2]

A pharmaceutical composition was produced in the same manner as in Example 2 except that l-carrageenan, methacrylic acid copolymer S, and SNAC were not added to the granulation solution.
In the produced pharmaceutical composition, the content of the core particle was 31.3% by mass with respect to the total mass of the pharmaceutical composition, and the content of the coating particle layer was 68.7% by mass with respect to the total mass of the pharmaceutical composition. In addition, the average particle diameter of the pharmaceutical compositions was 300 μm, and the average particle diameter of the core particles was 204 μm.

[Comparative Example 3]

FD-4 and SNAC were mixed to produce a pharmaceutical composition.

[Comparative Example 4]

The FD-4 was used as it was.
The compositions of the pharmaceutical compositions and the like of Examples 1 and 2, Examples A and B, and Comparative Examples 1 to 4 are shown in Table 1 below.

[Table 1]

| | Core particle | | | | | | Coating particle layer | | SNAC/FD-4 |
|---|---|---|---|---|---|---|---|---|---|
| | Medicinal ingredient | Absorption enhancing agent | Thickener | | | Enteric polymer | Coating particle | | |
| | FD-4 (% by mass) | SNAC (% by mass) | Glyceryl monostearate (% by mass) | Gelatin (% by mass) | Carrageenan (% by mass) | Methacrylic acid copolymer S (% by mass) | Bentonite (% by mass) | Corn starch (% by mass) | |
| Example 1 | 0.3 | 0.6 | 81.6 | 0 | 0 | 0 | 17.5 | 0 | 2.0 |
| Example 2 | 0.3 | 0.6 | 0 | 21.8 | 0.2 | 8.4 | 68.7 | 0 | 2.0 |
| Example A | 3.0 | 6.0 | 0 | 14.2 | 0.1 | 5.8 | 70.9 | 0 | 2.0 |
| Example B | 2.8 | 5.6 | 0 | 13.2 | 0.1 | 5.4 | 0 | 72.9 | 2.0 |
| Comparative Example 1 | 0.3 | 0 | 82.2 | 0 | 0 | 0 | 17.5 | 0 | - |
| Comparative Example 2 | 0.3 | 0 | 0 | 31 | 0 | 0 | 68.7 | 0 | - |
| Comparative Example 3 | 33.3 | 66.7 | 0 | 0 | 0 | 0 | 0 | 0 | 2.0 |
| Comparative Example 4 | 100 | 0 | 0 | 0 | 0 | 0 | 0 | 0 | - |

[Evaluation]

The pharmaceutical compositions and the like produced in Examples 1 and 2, Examples A and B, and Comparative Examples 1 to 4 were subjected to various evaluations.

(Area Under Curve (AUC))

An area under the curve (AUC) at the time of oral administration was evaluated for the pharmaceutical compositions and the like of Examples 1 and 2 and Comparative Examples 1 to 4.

A sample was prepared by dissolving or suspending the pharmaceutical composition and the like of Examples 1 and 2 and Comparative Examples 1 to 4 in water. Next, FD-4 was orally administered to Wistar rats using an oral sonde so that the dosage was 0.3 mg/kg. After the administration of the sample, blood was collected six times in total until six hours after the administration of the sample, and plasma was fractionated. The concentration of FD-4 in the fractionated plasma was measured using a fluorescence spectrophotometer. From the obtained results of the concentration of FD-4 in the plasma at each elapsed time, AUC (area under the curve) was calculated by a linear trapezoidal method. The obtained results are shown in Table 2-1 below.

[Table 2-1]

| | Time (hr) | | | | | | AUC (ng·hr/mL) |
|---|---|---|---|---|---|---|---|
| | 0.25 | 0.5 | 1 | 2 | 4 | 6 | |
| Example 1 | 19.9 | 17.6 | 16.4 | 14.8 | 16.2 | 13.2 | 91.6 |
| Example 2 | 6.8 | 11.8 | 11.7 | 29.1 | 12.1 | 10.4 | 78.1 |
| Comparative Example 1 | 6.1 | 5.0 | 5.6 | 5.6 | 6.6 | 6.0 | 35.1 |
| Comparative Example 2 | 12.1 | 10.4 | 9.4 | 7.3 | 7.6 | 6.7 | 42.8 |
| Comparative Example 3 | 15.5 | 12.2 | 9.0 | 7.6 | 7.9 | 6.4 | 48.8 |
| Comparative Example 4 | 3.0 | 4.1 | 3.0 | 4.2 | 4.8 | 4.0 | 24.3 |

From the results in Table 2-1, the pharmaceutical compositions of Examples 1 and 2 were found to have a high AUC. In particular, the AUCs of the pharmaceutical compositions of Examples 1 and 2 were respectively 3.8 times and 3.2 times higher than that of Comparative Example 4.

It is noted that, the AUCs of the pharmaceutical compositions of Comparative Example 1 and Comparative Example 2 were respectively 1.4 times and 1.8 times higher than that of Comparative Example 4. Further, the AUC of the pharmaceutical composition of Comparative Example 3 was 2.0 times higher than that of Comparative Example 4.

(Increase Ratio (ER) of AUC in Consideration of Dosage)

For the pharmaceutical compositions and the like of Examples 1 and 2, Examples A and B, and Comparative Examples 1 to 3, the increase ratio (ER) based on the result of Comparative Example 4 was evaluated.

1. Acquisition of Reference Data of Standard Preparation

The data of the "area under the curve (AUC)" of Comparative Example 4, that is, AUCs (24.3 ng·h/mL) when FD-4 (dosage s: 0.3 mg/kg) was orally administered to a rat in its original form was used as reference data of a standard preparation.

It is noted that, in Comparative Example 4, AUC in a case where the dosage of FD-4 was set to 5.0 mg/kg was measured in the same manner as in the "area under the curve (AUC)", and it was 390.5 ng·h/mL. The AUC (AUC/dosage) per dosage was almost the same between a case in which dosage was 5.0 mg/kg and a case in which dosage was 0.3 mg/kg, and was 0.96 when ER to be described later was calculated for a case in which dosage was 5.0 mg/kg. That is, it has been confirmed that when the FD-4 is used as it is, the ER does not increase in correlation with the dosage.

2. Acquisition of Target Data of Test Preparation

In Examples 1 and 2 and Comparative Examples 1 to 3, data of the "area under the curve (AUC)" was used as data when the dosage (dosage t) of the FD-4 was set to 0.3 mg/kg.

In Examples A and B, AUC in a case where the dosage (dosage t) of FD-4 was set to 1.4 mg/kg or 1.5 mg/kg was measured in the same manner as in the "area under the curve (AUC)".

As a result, target data, that is, AUCt, when the pharmaceutical compositions and the like of Examples 1 and 2, Examples A and B, and Comparative Examples 1 to 3 were orally administered was acquired.

3. Calculation of Increase Ratio (ER)

The increase ratio (ER) based on the reference data of the standard preparation (Comparative Example 4) was calculated by the following formula.

$$\mathrm{ER} = \frac{\mathrm{AUCt(ng \cdot h/mL)/Dosage\ t\ (mg/kg)}}{\mathrm{AUCs(ng \cdot h/mL)/Dosage\ s\ (mg/kg)}}$$

It is noted that "AUCt" is AUC (ng·h/mL) of the test preparation, and "dosage t" is dosage (mg/kg) of the test preparation (target data of the test preparation obtained in the above 2).

In addition, "AUCs" is the AUC (24.3 ng·h/mL) of the standard preparation, and "dosage s" is the dosage (0.3 mg/kg) of the standard preparation (reference data of the standard preparation acquired in the above 1).

The obtained results are shown in Table 2-2 below.

[Table 2-2]

| | FD-4 Dosage (mg/kg) | SNAC Dosage (mg/kg) | ER |
|---|---|---|---|
| Example 1 | 0.3 | 0.6 | 3.8 |
| Example 2 | 0.3 | 0.6 | 3.2 |
| Example A | 1.5 | 3.0 | 12.1 |
| Example B | 1.4 | 2.8 | 3.7 |
| Comparative Example 1 | 0.3 | 0 | 1.4 |
| Comparative Example 2 | 0.3 | 0 | 1.8 |
| Comparative Example 3 | 0.3 | 0.6 | 2.0 |

From the results in Table 2-2, it was found that the pharmaceutical compositions of Examples 1 and 2 and Examples A and B have high ER.

[Example 3]

A granulation solution was prepared. Gelatin and l-carrageenan each serving as a thickener, and water were heated and dissolved at 90°C, then the temperature was lowered to 70°C, and then cyanocobalamin and sodium caprate serving as an absorption enhancing agent were added, thereby preparing a granulation solution.

A pharmaceutical composition having a core particle derived from a granulation solution and a coating particle layer of corn starch was produced in the same manner as in Example 1 except that corn starch was used instead of bentonite.

It is noted that, in the produced pharmaceutical composition, the content of the core particle was 70.0% by mass with respect to the total mass of the pharmaceutical composition, and the content of the coating particle layer was 30.0% by mass with respect to the total mass of the pharmaceutical composition. In addition, the average particle diameter of the pharmaceutical compositions was 300 μm, and the average particle diameter of the core particles was 266 μm.

[Example 4]

A granulation solution was prepared in the same manner as in Example 3 except that the contents of gelatin, l-carrageenan, and sodium caprate were changed.

A pharmaceutical composition having a core particle derived from a granulation solution and a coating particle layer of corn starch was produced in the same manner as in Example 1 except that corn starch was used instead of bentonite.

It is noted that, in the produced pharmaceutical composition, the content of the core particle was 70.0% by mass

with respect to the total mass of the pharmaceutical composition, and the content of the coating particle layer was 30.0% by mass with respect to the total mass of the pharmaceutical composition. In addition, the average particle diameter of the pharmaceutical compositions was 300 μm, and the average particle diameter of the core particles was 266 μm.

[Examples 5 to 9]

A granulation solution was prepared in the same manner as in Example 3 except that the contents of gelatin, l-carrageenan, cyanocobalamin, and sodium caprate were changed as shown in Table 3.

A pharmaceutical composition having a core particle derived from the granulation solution and a coating particle layer of bentonite was produced in the same manner as in Example 1.

It is noted that, in the produced pharmaceutical composition, the content of the core particle was 20.0% by mass with respect to the total mass of the pharmaceutical composition, and the content of the coating particle layer was 80.0% by mass with respect to the total mass of the pharmaceutical composition. In addition, the average particle diameter of the pharmaceutical compositions was 300 μm, and the average particle diameter of the core particles was 175 μm.

The composition of the pharmaceutical composition of Examples 3 to 9 is shown in Table 3 below.

[Table 3]

| | Core particle | | | | Coating particle layer | | Sodium caprate /Cyanocobalamin |
|---|---|---|---|---|---|---|---|
| | Medicinal ingredient | Absorption enhancing agent | Thickener | | Coating particle | | |
| | Cyanocobalamin (% by mass) | Sodium caprate (% by mass) | Gelatin (% by mass) | Carrageenan (% by mass) | Corn starch (% by mass) | Bentonite (% by mass) | |
| Example 3 | 1 | 6.7 | 61.7 | 0.6 | 30 | 0 | 6.7 |
| Example 4 | 1 | 16.6 | 51.9 | 0.5 | 30 | 0 | 16.6 |
| Example 5 | 0.3 | 5 | 14.6 | 0.1 | 0 | 80 | 16.5 |
| Example 6 | 0.2 | 10.1 | 9.6 | 0.1 | 0 | 80 | 50.5 |
| Example 7 | 0.2 | 13.6 | 6.1 | 0.1 | 0 | 80 | 68 |
| Example 8 | 0.1 | 12.2 | 7.6 | 0.1 | 0 | 80 | 122.3 |
| Example 9 | 0.03 | 13.6 | 6.3 | 0.1 | 0 | 80 | 451.7 |

[Elution Test]

Gastric solubility and small intestine enteric property were evaluated for the pharmaceutical composition of Examples 3 to 9.

(Gastric Solubility Evaluation)

A first liquid for an elution test (hereinafter, also referred to as "liquid I") was prepared by dissolving 2.0 g of sodium chloride in 7.0 mL of hydrochloric acid and water to make 1000 mL. It is noted that the pH of liquid I was 1.2, which was equivalent to the pH in the stomach.

Using an apparatus for a paddle method described in an elution test method (6.10) of the Japanese Pharmacopoeia, 18th Edition, 200 mg of a pharmaceutical composition was added to liquid I stirred at 50 rpm at 37°C, and liquid I

after 120 minutes was fractionated. The amount of cyanocobalamin in liquid I was measured by high performance liquid chromatography (HPLC), and the elution rate (%) of cyanocobalamin eluted from the pharmaceutical composition into liquid I (elution rate of liquid I) was calculated. It is noted that the measurement conditions of HPLC are described as follows.

Apparatus: Prominence (manufactured by SHIMADZU CORPORATION)
Column: Inertsil ODS (manufactured by GL Sciences Inc.)
Developing solvent: water : methanol : acetic acid = 750 : 250 : 10
Measurement sample: 1 mL of liquid I and 1 mL of developing solvent were mixed.
Quantification method: performed by ultraviolet-visible spectrophotometry (wavelength: 360 nm)

(Evaluation of Small Intestine Enteric Property)

A second liquid for an elution test (hereinafter, also referred to as "liquid II") was prepared by adding 1 volume of water to 1 volume of a phosphate buffer liquid having a pH of 6.8. It is noted that the pH of liquid II was 6.8, which was equivalent to the pH in the small intestine.

The elution rate (%) of cyanocobalamin eluted from the pharmaceutical composition into liquid II (elution rate of liquid II) was calculated in the same manner as in the evaluation of gastric solubility.

The obtained results are shown in Table 4 below.

[Table 4]

| | Elution rate of liquid I (%) | Elution rate of liquid II (%) |
|---|---|---|
| Example 3 | 85 | 100 |
| Example 4 | 84 | 100 |
| Example 5 | 0 | 100 |
| Example 6 | 0 | 100 |
| Example 7 | 0 | 100 |
| Example 8 | 0 | 100 |
| Example 9 | 0 | 100 |

From the results in Table 4, it was found that Examples 5 to 9 show enteric properties. In addition, it was found that, even when the content (absorption enhancing agent/medicinal ingredient) of the absorption enhancing agent with respect to 1 part by mass of the medicinal ingredient was high, the pharmaceutical composition was not disintegrated with liquid I, and has high form retention.

## Claims

1. A pharmaceutical composition comprising:

    a core particle containing a medicinal ingredient and an absorption enhancing agent; and
    a coating particle layer coating the core particle.

2. The pharmaceutical composition according to claim 1, wherein the core particle further contains a thickener.

3. The pharmaceutical composition according to claim 1, wherein the core particle further contains a hydrophobic thickener.

4. The pharmaceutical composition according to claim 2, wherein the thickener contains at least one selected from a group consisting of carrageenan, gelatin, glyceryl monostearate, agar, polyethylene glycol, methyl cellulose, hydroxypropyl methyl cellulose, hydroxypropyl cellulose, an aminoalkyl methacrylate copolymer, and an ammonioalkyl methacrylate copolymer.

5. The pharmaceutical composition according to claim 1, wherein the coating particle layer contains at least one selected from a group consisting of bentonite, ethyl cellulose, corn starch, rice starch, wheat starch, potato starch, calcium stearate, magnesium carbonate, sodium carboxymethyl starch having a low degree of substitution, sodium starch glycolate, anhydrous silicic acid, magnesium silicate, diatomaceous earth, zeolite, silicon dioxide, powdered agar, croscarmellose sodium, crospovidone, and talc.

6. The pharmaceutical composition according to claim 1, wherein the core particle further contains an enteric polymer.

7. The pharmaceutical composition according to claim 1, wherein the absorption enhancing agent contains at least one selected from a group consisting of sodium caprate, sodium caprylate, SNAC, cell membrane penetrating peptide, C-CPE, Angubindin-1, polyoxyethylene alkyl ethers, sodium lauryl sulfate, saponin, chitosan, cyclodextrin, alkyl saccharide, sucrose fatty acid ester, N-acyl amino acid, N-acyl taurine, glycocholic acid, taurocholic acid, deoxycholic acid, ethylenediaminetetraacetic acid (EDTA), sodium salicylate, sodium laurate, tryptophan, arginine, and fatty acids having 8 to 30 carbon atoms.

8. The pharmaceutical composition according to claim 1, wherein a content of the absorption enhancing agent is 1 to 20% by mass with respect to a total mass of the pharmaceutical composition.

9. The pharmaceutical composition according to claim 1, wherein the medicinal ingredient is a polymer ingredient.

10. The pharmaceutical composition according to claim 1, wherein a content of the absorption enhancing agent is 1 to 500 parts by mass with respect to 1 part by mass of the medicinal ingredient.

11. A method for producing the pharmaceutical composition according to any one of claims 1 to 10, comprising a granulation step of inputting a droplet of a granulation solution containing a medicinal ingredient and an absorption enhancing agent into a fluidized bed in which coating particles flow, and performing granulation by attaching the coating particles to a surface of the droplet.

[FIG. 1]

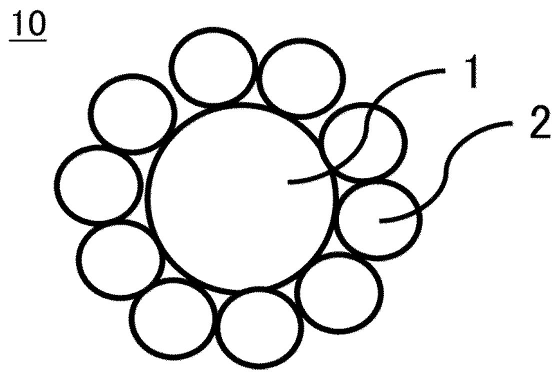

## INTERNATIONAL SEARCH REPORT

International application No.
**PCT/JP2023/023170**

### A. CLASSIFICATION OF SUBJECT MATTER

***A61K 9/14***(2006.01)i; ***A61K 9/20***(2006.01)i; ***A61K 31/721***(2006.01)i; ***A61K 47/02***(2006.01)i; ***A61K 47/04***(2006.01)i; ***A61K 47/10***(2017.01)i; ***A61K 47/12***(2006.01)i; ***A61K 47/14***(2017.01)i; ***A61K 47/18***(2017.01)i; ***A61K 47/20***(2006.01)i; ***A61K 47/22***(2006.01)i; ***A61K 47/28***(2006.01)i; ***A61K 47/32***(2006.01)i; ***A61K 47/34***(2017.01)i; ***A61K 47/36***(2006.01)i; ***A61K 47/38***(2006.01)i; ***A61K 47/40***(2006.01)i; ***A61K 47/42***(2017.01)i; ***A61P 43/00***(2006.01)i

FI: A61K9/14; A61K9/20; A61K31/721; A61K47/02; A61K47/04; A61K47/10; A61K47/12; A61K47/14; A61K47/18; A61K47/20; A61K47/22; A61K47/28; A61K47/32; A61K47/34; A61K47/36; A61K47/38; A61K47/40; A61K47/42; A61P43/00

According to International Patent Classification (IPC) or to both national classification and IPC

### B. FIELDS SEARCHED

Minimum documentation searched (classification system followed by classification symbols)

A61K9/14; A61K9/20; A61K31/721; A61K47/02; A61K47/04; A61K47/10; A61K47/12; A61K47/14; A61K47/18; A61K47/20; A61K47/22; A61K47/28; A61K47/32; A61K47/34; A61K47/36; A61K47/38; A61K47/40; A61K47/42; A61P43/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X | JP 2008-522989 A (EVONIK ROEHM GMBH) 03 July 2008 (2008-07-03) claims, paragraph [0021], examples 2-5 | 1-11 |
| X | JP 2016-523250 A (RAPTOR PHARMACEUTICALS INC) 08 August 2016 (2016-08-08) claims, example 1 | 1-2, 4-8, 10-11 |
| X | JP 2008-500339 A (THE PROCTER & GAMBLE COMPANY) 10 January 2008 (2008-01-10) claims, paragraphs [0007], [0091], example V | 1-8, 10-11 |
| X | JP 2018-505172 A (NOVO NORDISK A/S) 22 February 2018 (2018-02-22) claims, paragraphs [0018]-[0100], examples | 1, 5-7, 9-11 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 September 2023** | **12 September 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2023/023170**

**C. DOCUMENTS CONSIDERED TO BE RELEVANT**

| Category* | Citation of document, with indication, where appropriate, of the relevant passages | Relevant to claim No. |
|---|---|---|
| X | JP 2011-519915 A (ORAMED LTD) 14 July 2011 (2011-07-14) claims, paragraphs [0041]-[0042], [0078], example 4 | 1-11 |
| A | 民輪英之ほか, バイオ医薬に活用される粘膜吸収促進剤の開発, Drug Delivery System. 2020, 35-1, pp. 10-19 2. Transdermal absorption enhancer, 2-1.Sodium caprate, 2-2. SNAC (sodium N-[8-(2-hydroxybenzoyl)amino]caprylate), 3. CPP expected to be used as a novel absorption enhancer, Usefulness of tryptophan, table 1, (TAMIWA, Hideyuki et al. Development of mucosal absorption enhancers used biopharmaceuticals.) | 1-11 |

Form PCT/ISA/210 (second sheet) (January 2015)

**INTERNATIONAL SEARCH REPORT**
**Information on patent family members**

International application No.
**PCT/JP2023/023170**

| Patent document cited in search report | Publication date (day/month/year) | Patent family member(s) | Publication date (day/month/year) |
|---|---|---|---|
| JP 2008-522989 A | 03 July 2008 | US 2009/0280183 A1<br>claims, paragraph [0029], examples 2-5<br>CN 101060835 A<br>KR 10-2007-0085894 A | |
| JP 2016-523250 A | 08 August 2016 | WO 2014/204881 A1<br>claims, example 1<br>US 2014/0370085 A1<br>CN 105492000 A<br>KR 10-2016-0045053 A | |
| JP 2008-500339 A | 10 January 2008 | US 2005/0260262 A1<br>claims, paragraphs [0010], [0110], example V<br>CN 102225047 A<br>KR 10-2007-0111551 A | |
| JP 2018-505172 A | 22 February 2018 | US 2018/0000903 A1<br>claims, paragraphs [0018]-[0019], examples<br>CN 107205948 A | |
| JP 2011-519915 A | 14 July 2011 | US 2011/0046053 A1<br>claims, paragraphs [0043]-[0044], [0081], example 4<br>CN 102026646 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2016519128 A
- JP 2019073459 A